# EUROPÄISCHE PATENTSCHRIFT

(11) **EP 4 499 620 B1**
(45) Veröffentlichungstag und Bekanntmachung des Hinweises auf die Patenterteilung: **18.02.2026**
(21) Anmeldenummer: 23713121.4
(22) Anmeldetag: 23.03.2023
(51) Int. Cl.: C07D 307/20, C07C 211/27, C07D 307/22, C07D 307/24

(54) **VERFAHREN ZUR HERSTELLUNG VON CIS-4-AMINOTETRAHYDROFURAN-2-CARBONSÄUREESTERN**
METHOD FOR THE PREPARATION OF CIS-4-AMINOTETRAHYDROFURAN-2-CARBOXYLATES
PROCÉDÉ DE PRODUCTION D'ESTERS DE CIS-4-AMINOTÉTRAHYDROFURANE-2-CARBONATE

(30) Priorität: 28.03.2022 EP 22164857
(43) Veröffentlichungstag der Anmeldung: 05.02.2025
(73) Patentinhaber: Bayer Aktiengesellschaft, 51373 Leverkusen (DE)
(72) Erfinder: BROHM, Dirk, 51373 Leverkusen (DE); REMBIAK, Andreas, 51373 Leverkusen (DE); LISHCHYNSKYI, Anton, 51373 Leverkusen (DE)
(74) Vertreter: BIP Patents
(86) Internationale Anmeldenummer: PCT/EP2023/057452
(87) Internationale Veröffentlichungsnummer: WO 2023/186692

(56) Entgegenhaltungen:
- WO-A1-2021/170464
- WO-A2-2012/137982

## Beschreibung

Die vorliegende Erfindung betrifft ein neues Verfahren zur Herstellung von cis-4-Aminotetrahydrofuran-2-carbonsäureester der allgemeinen Formel (I) und deren Salze.

Cis-4-Aminotetrahydrofuran-2-carbonsäuremethylester Hydrochlorid (CAS 1304126-28-0) der allgemeinen Formel (I) (mit R¹=Me) ist ein wichtiger Baustein zur Synthese von Pflanzenschutzmitteln (WO 2012/130798, WO 2021/170464).

Die erste publizierte Syntheseroute ist von Walker et al. (Synthesis 2011, 7, 1113-1119). Es wird 4-(*tert-*Butoxycarbonylamino)furan-2-carbonsäuremethylester (CAS 1170719-58-0, Wolter et al., Org. Lett. 2009, 11, 2804) als Vorstufe verwendet. 4-(*tert*-Butoxycarbonylamino)furan-2-carbonsäuremethylester ist allerdings aufgrund der hohen Kosten der Reagenzien zur Herstellung, der geringen Ausbeuten, Sicherheitsrisiken und Phosphatabfällen im großtechnischen Maßstab nicht wirtschaftlich herstellbar. Beispielsweise sind dafür eine Lithiierung mit sec-Butyllithium (34% Ausbeute) und eine Curtius Umlagerung mit DPPA (Diphenylphosphonazid) notwendig. Daher ist diese Route zur Herstellung von cis-4-Aminotetrahydrofuran-2-carbonsäureestern im industriellen Maßstab nicht machbar.

Auch die Synthese über 4-Bromfuran-2-carbonsäuremethylester (CAS 58235-80-6, WO 2021/170464) ist aufgrund der Bromierung und der anschließenden ineffizienten Kupplung mit tert-Butylcarbamat (25% Ausbeute, WO 2021/170464) kostenintensiv und führt zu großen Mengen an Abfällen.

Daher besteht ein hoher Bedarf an neuen, kostengünstigen und umweltfreundlichen Prozessen zur Synthese von cis-4-Aminotetrahydrofuran-2-carbonsäureestern, wodurch dann auch die entsprechenden Pflanzenschutzmittel im großtechnischen Maßstab hergestellt werden können.

In WO 2012/137982 wird die reduktive Aminierung von 4-Oxotetrahydrofuran-3-carbonsäuremethylester mit Benzylamin beschrieben. Bei diesem Substitutionsmuster ist es möglich, das Keton mit dem Benzylamin zunächst zu einem Enamin zu entwässern, das über die Konjugation zum Ester stabilisiert ist. Die anschließende Reduktion der Doppelbindung wurde dann mit Natriumtriacetoxyborhydrid durchgeführt.

Diese Erfindung beschreibt ein neues Verfahren zur Herstellung von cis-4-Aminotetrahydrofuran-2-carbonsäureestern ausgehend von 4-Oxotetrahydrofuran-2-carbonsäureestern der allgemeinen Formel (II). Die Herstellung dieser Ausgangssubstanz wird beispielsweise in WO 2016/205633 beschrieben.

Im Lichte des zuvor beschriebenen Standes der Technik liegt der vorliegenden Erfindung die Aufgabe zugrunde, ein Verfahren zur Herstellung der genannten Verbindungen zu finden, das kostengünstig ist und großtechnisch eingesetzt werden kann. Auch ist es erstrebenswert, diese Verbindungen in hoher Ausbeute und in hoher Reinheit zu erhalten, so dass sie keiner weiteren komplexen Aufreinigung unterzogen werden müssen.

Die zuvor beschriebene Aufgabe - einfache, kostengünstige und großtechnische Herstellung - wird gelöst durch ein Verfahren zur Herstellung von Verbindungen der allgemeinen Formel (I) oder deren Salze 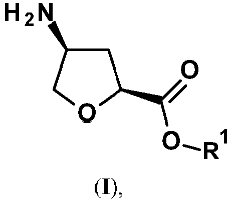 worin
- R¹: für (C₁-C₆)Alkyl oder (C₃-C₆)Cycloalkyl steht,
dadurch gekennzeichnet, dass in einem ersten Schritt Verbindungen der allgemeinen Formel (II) 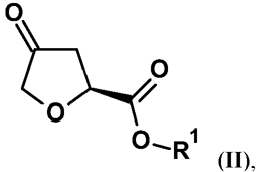 worin
- R¹: wie oben definiert ist
mit einem Amin der allgemeinen Formel (III) oder deren Salze 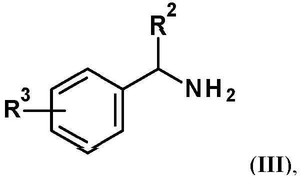 worin
- R²: für H, (C₁-C₄)Alkyl, unsubstituiertes oder substituiertes Phenyl steht,
- R³: für H, Halogen, (C₁-C₄)Alkyl, (C₁-C₄)Alkoxy steht,
in Gegenwart von H₂ und einem Katalysator in einem Lösungsmittel, ausgewählt aus R'OH, R'OH/Toluol, 2-PrOH, 2-BuOH oder t-Amylalkohol, wobei R¹ wie oben definiert ist zu Verbindungen der allgemeinen Formel (IV) oder deren Salze reagieren 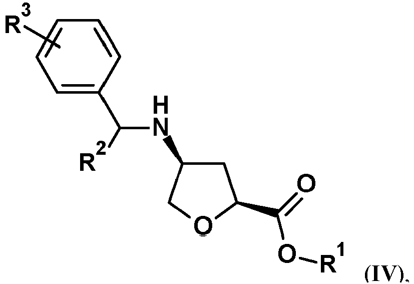 wobei R¹, R² und R³ wie oben definiert sind,
und diese anschließend in Gegenwart von H₂ und einem Katalysator in einem Lösungsmittel, ausgewählt aus R'OH, R'OH/Toluol, 2-PrOH, 2-BuOH oder t-Amylalkohol, wobei R¹ wie oben definiert ist, zu Verbindungen der allgemeinen Formel (I) oder deren Salze umgesetzt werden.

Bevorzugte Restedefinitionen für die Verbindungen der allgemeinen Formeln **(I), (II), (III)** und **(IV)** oder deren Salze sind die folgenden:
- R¹: steht für (C₁-C₆)Alkyl,
- R²: steht für H, Phenyl, Methyl,
- R³: steht für H, F in para-Position des Phenylrings, Methyl-O- in para-Position des Phenylrings, Methyl in ortho-Position des Phenylrings.

Besonders bevorzugte Restedefinitionen für die Verbindungen der allgemeinen Formeln **(I), (II), (III)** und **(IV)** oder deren Salze sind die folgenden:
- R¹: steht für CH₃,
- R²: steht für H,
- R³: steht für H oder Methyl in ortho-Position des Phenylrings.

Die Reaktionsfolge zur Herstellung von Verbindungen der Formel **(I)** oder deren Salze ist im Schema 1 dargestellt.

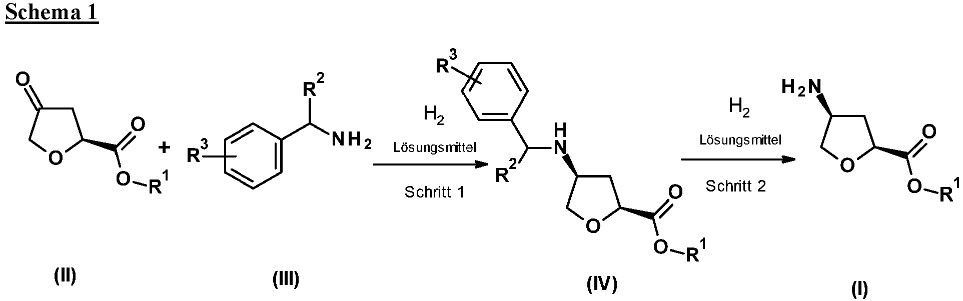

Die Verbindungen der allgemeinen Formel **(II)** reagieren im ersten Reaktionsschritt mit Verbindungen der allgemeinen Formel **(III)** oder deren Salze (ggf. handelt es sich auch um Mischungen der Verbindungen der allgemeinen Formel **(III)** und deren Salze) Wasserstoff und einem Katalysator in einem Lösungsmittel zu Verbindungen der allgemeinen Formel **(IV)** oder deren Salze.

Die dabei gewonnenen Amine lassen sich in einem weiteren Schritt zu Salzen von cis-4-Aminotetrahydrofuran-2-carbonsäureester der allgemeinen Formel **(I)** oder deren Salze unter Verwendung eines Katalysators und Wasserstoff in einem Lösungsmittel umsetzen.

Beide Schritte können außerdem unter Verwendung eines Katalysators oder einer Mischung von Katalysatoren und Wasserstoff in einem einstufigen Verfahren kombiniert werden.

### Schritt 1:

Die Verbindungen der allgemeinen Formeln **(III)** und **(IV)** können als freie Amine oder in Form ihrer Salze beim Einsatz von Säuren vorliegen, bevorzugt ist die Salzform. Bevorzugt werden folgende Säuren zur Salzbildung mit Verbindungen der allgemeinen Formel **(II)** und/oder als Zusatz eingesetzt: HCl, H₂SO₄, MsOH, TfOH, TFA. Besonders bevorzugt ist HCl.

Der Druck bei der Reaktion beträgt 1 bis 100 bar, bevorzugt 1 bis 50 bar, besonders bevorzugt 20 bis 50 bar.

Die Temperatur bei der Reaktion beträgt 0°C bis 100 °C, bevorzugt 4°C bis 80°C, besonders bevorzugt 20 bis 40°C.

Als Katalysatoren können Raney-Nickel, Raney-Cobalt, Pt/C und Pt/Alox eingesetzt werden, bevorzugt Raney-Nickel und Pt/C, besonders bevorzugt Pt/C.

Die Katalysatoren werden in einer Menge von 0,1-10 Gew.-% in Bezug auf die Verbindungen der allgemeinen Formel **(II)** eingesetzt, bevorzugt werden 0,5-5 Gew.-% eingesetzt. Die Menge der eingesetzten Katalysatoren wird auf die Trockenmasse der Katalysatoren berechnet. Die Katalysatoren können trocken oder wasserfeucht eingesetzt werden.

Dem Reaktionsgemisch können zusätzlich Lewis-Säuren hinzugefügt werden, wie z.B.: ZnCl₂, LiCl, MgCl₂, CeCl₃, CoCl₂, AlCl₃, FeCl₃, CaCl₂, Ti(iOPr)₄, B(OMe)₃, bevorzugt ist die Durchführung der Reaktion ohne Lewis-Säure Additiv.

Geeignete Lösungsmittel und Lösungsmittelgemische sind z.B.: R¹OH, R¹OH/Toluol, 2-PrOH, 2-BuOH, t-Amylalkohol; bevorzugt sind R¹OH und R¹OH/Toluol.

Das molare Verhältnis der Verbindungen der allgemeinen Formel **(II)** und **(III)** liegt im Bereich von etwa 0,5 bis 2, bevorzugt 0,9 bis 1,1, besonders bevorzugt 1.

Fallen die Verbindungen der allgemeinen Formel (I) in Form ihrer Salze an, z.B. als Hydrochlorid, kann die salzfreie Form gewonnen werden, indem das Salz mit einer Base, z.B. Triethylamin, behandelt wird.

### Schritt 2:

Die Verbindungen der allgemeinen Formeln **(I)** und **(IV)** können als freie Amine oder in Form deren Salze mit Säuren vorliegen, bevorzugt ist die Salzform. Bevorzugt werden folgende Säuren zur Salzbildung mit Verbindungen der Formel (II) und/oder als Zusatz eingesetzt: HCl, H₂SO₄, MsOH, TfOH, TFA. Besonders bevorzugt ist HCl.

Der Druck bei der Reaktion beträgt 1 bis 100 bar, bevorzugt 1 bis 20 bar, besonders bevorzugt 1 bis 5 bar.

Die Temperatur bei der Reaktion beträgt 0°C bis 100 °C, bevorzugt 20°C bis 80°C, besonders bevorzugt 40 bis 80°C.

Als Katalysatoren können Pd/C und Pd/Alox eingesetzt werden; bevorzugt: Pd/C.

Die Katalysatoren werden mit einer Menge von 0,1-10 Gew.-% in Bezug auf die Verbindungen der Formel **(II)** eingesetzt; bevorzugt werden 0,1-2 Gew.-% eingesetzt. Die Menge der eingesetzten Katalysatoren wird auf die Trockenmasse der Katalysatoren berechnet. Die Katalysatoren können trocken oder wasserfeucht eingesetzt werden. Wasserfeuchte Katalysatoren können vor der Reaktion mit trockenem Lösungsmittel unter Inertgas-Atmosphäre gewaschen werden, um anhaftendes Wasser zu entfernen. Alternativ kann das Wasser durch azeotrope Destillation mit einem geeigneten Lösungsmittel entfernt werden. Für die azeotrope Destillation kann beispielsweise Toluol eingesetzt werden.

Der Katalysator kann einmal oder bevorzugt auch mehrfach wiederverwendet bzw. eingesetzt werden. Eine Reaktivierung des Katalysators kann, beispielsweise durch geeignete Wäschen, vorteilhaft für dessen Wiederverwendung sein, beispielsweise durch Wäschen mit Methanol oder saurem Methanol. Geeignete Lösungsmittel und Lösungsmittelgemische sind z.B.: R¹OH, R¹OH/Toluol; bevorzugt sind MeOH und MeOH/Toluol.

Fallen die Verbindungen der allgemeinen Formel **(I)** in Form ihrer Salze an, z.B. als Hydrochlorid, kann die salzfreie Form gewonnen werden, indem das Salz mit einer Base, z.B. Triethylamin, behandelt wird.

Überraschenderweise wurde nun gefunden, dass sich 4-Oxotetrahydrofuran-2-carbonsäureester mit ggf. substituierten Phenylmethylaminen der allgemeinen Formel **(III)** oder deren Salzen in hoher *cis-*Selektivität und hoher Ausbeute unter Verwendung von Wasserstoff und einem Katalysator in einem Lösungsmittel in einer reduktiven Aminierung zu den entsprechenden N-substituierten cis-4-Aminotetrahydrofuran-2-carbonsäureestern der allgemeinen Formel **(IV)** direkt umsetzen lassen. Da aufgrund des anderen Substitutionsmusters keine Konjugation und Stabilisierung des Enamins durch den Ester wie in WO 2012/137982 stattfinden kann, war dies nicht naheliegend. Beispielsweise konnte aus 4-Oxotetrahydrofuran-2-carbonsäuremethylester ein entsprechendes Enamin analog wie in WO 2012/137982 bisher nicht isoliert werden. Unter diesen Bedingungen entsteht dagegen hauptsächlich das Benzylamid des Esters. Des Weiteren ist die Reduktion mit Wasserstoff an einem Katalysator notwendig, um die gewünschte cis-Selektivität zu erhalten. Die reduktive Aminierung mit Ammoniak oder Ammoniumsalzen war auch nicht erfolgreich.

### Erläuterung der Verfahren und Zwischenprodukte

### Beispiele

Die vorliegende Erfindung wird anhand der nachfolgenden Beispiele näher erläutert, ohne die Erfindung dabei auf diese einzuschränken.

### Messverfahren

Die Produkte wurden mittels ¹H-NMR Spektroskopie, Standard HPLC der Fa. Agilent und/oder GC/MS (Gas Chromatography Mass Spectrometry) charakterisiert.

Die NMR-Spektren wurden mit einem Bruker AV III 600 gemessen.

Die GC/MS Proben wurden mit einem Shimadzu GCMS-QP-2010-Ultra gemessen, das mit einem zusätzlichen FID (Flammen-Ionisations-Detektor) gekoppelt ist. Dazu wurden die Proben zunächst eingedampft und dann 1-10 mg trockene Probe zur Silylierung mit 250 µL N-Methyl-N-trimethylsilyltrifluoracetamid versetzt. Nach 1-5 Minuten Reaktionszeit wurden die Proben mit 1 ml Acetonitril verdünnt und gemessen.

### Erfindungsgemäßer Schritt 1

### Beispiel 1

### rac-cis-4-(Benzylamino)tetrahydrofuran-2-carbonsäuremethylesterhydrochlorid

Zu einer Lösung von 49,3 g (343 mmol) Benzylaminhydrochlorid und 250 g Methanol in einem 600 ml Autoklaven wurden 50 g (343 mmol, 1 eq) 4-Oxotetrahydrofuran-2-carbonsäuremethylester und 5 g (0,44 mmol) 5% Pt/C Katalysator (66% wasserfeucht) gegeben. Der Autoklav wurde dreimal mit 5 bar Argon gespült und dann das Gemisch unter 5 bar Wasserstoff 16 h bei 20°C und 600 U/min gerührt. Danach wurde der Autoklav entspannt, die Reaktionsmischung über eine Nutsche abgesaugt und der Rückstand mit Methanol gewaschen. Das Filtrat wurde unter reduziertem Druck auf 290 g eingeengt und mit 250 g Cyclopentylmethylether (CPME) versetzt. Die Lösung wurde unter reduziertem Druck auf 274 g eingeengt. Die entstandene Suspension wurde mit 50 g CPME und 15 g Methanol versetzt, dann 15 min bei 40°C sowie 1 h bei Raumtemperatur gerührt und dann über eine Nutsche abgesaugt. Der Rückstand wurde mit CPME/MeOH gewaschen und unter reduziertem Druck getrocknet.

GC/MS (m/z): 205 [M-CH₂O], 190, 176, 146, 132, 114, 106, 91, 82, 65.

¹H-NMR (DMSO-d6, 600 MHz): 2,1-2,2 (1H, m), 2,63-2,72 (1H, m), 3,69 (3H, s), 3,79-3,9 (1 H, m), 3,9-4,05 (2H, m), 4,07-4,21 (2H, m), 4,47-4,55 (1H, t), 7,35-7,5 (3H, m), 7,51-7,61 (2H, m), 9,5-9,7 (2H, br) ppm.
Reinheit (qNMR): 94%
Ausbeute: 56,4 g der Titelverbindung (57% d. Th.)

### Beispiele 2-13

Die folgenden Versuche sind analog zu der experimentellen Durchrührung zur Herstellung von Beispiel 1 durchgeführt worden. Die Ausbeute der Zielverbindung in Lösung wurde durch quantitative NMR Spektroskopie der Lösung bestimmt. Das Produkt wurde dabei nicht isoliert.

| Nr. | Verbindung Formel **(III)** | Katalysator [Gew.-%] | Lösungsmittel | Druck [bar] | Temperatur [°C] | Zeit [h] | cis/trans Verhältnis | Ausbeute, Lösung [%] |
|---|---|---|---|---|---|---|---|---|
| 2 | BnNH₂ | 10 | MeOH | 5 | 20 | 16 | 7,1 | 59 |
| 3 | BnNH₂ | 10 | 2-PrOH | 5 | 20 | 16 | 7,3 | 51 |
| 4 | BnNH₂ | 10 | 2-BuOH | 5 | 20 | 16 | 5,9 | 72 |
| 5 | BnNH₂ | 3,5 | tert-Amylalkohol | 5 | 22 | 16 | 7,8 | 78 |
| 6* | BnNH₂ | 3,0 | MeOH | 5 | 22 | 16 | 5,4 | 76 |
| 7 | BnNH₂*HCl | 10 | MeOH | 50 | 20 | 16 | 6,6 | 80 |
| 8 | BnNH₂*HCl | 10 | MeOH | 30 | 35 | 16 | 6,6 | 77 |
| 9 | BnNH₂*HCl | 10 | MeOH/Toluol (70/30) | 5 | 20 | 16 | 7,3 | 80 |
| 10 | BnNH₂*HCl | 5 | MeOH/Toluol (70/30) | 20 | 60 | 16 | 6,6 | 66 |
| 11** | BnNH₂*HCl | 10 | MeOH/Toluol (70/30) | 5 | 20 | 16 | 9,8 | 65 |
| 12 | BnNH₂*HCl + BnNH₂ | 3 | MeOH | 30 | 35 | 24 | 5,8 | 75 |
| 13*** | BnNH₂*HCl | 3 | MeOH | 30 | 35 | 24 | 5,0 | 76 |

| | | | | | | | | |
|---|---|---|---|---|---|---|---|---|
| * BnNH₂ über 4h dosiert.; *** 10 Gew.-% Zinkchlorid zugefügt; *** Verbindung **(II)** über 4h dosiert. | | | | | | | | |

### Beispiel 14

### rac-cis-4-(2-Methylbenzylamino)tetrahydrofuran-2-carbonsäuremethylesterhydrochlorid

Zu einer Lösung von 54,1 g (343 mmol) 2-Methylbenzylaminhydrochlorid und 300 ml Methanol in einem 600 ml Autoklaven wurden 50 g (343 mmol, 1 eq) 4-Oxotetrahydrofuran-2-carbonsäuremethylester und 5 g (0,44 mmol) 5% Pt/C Katalysator (66% wasserfeucht) gegeben. Der Autoklav wurde dreimal mit 5 bar Argon gespült und dann das Gemisch unter 5 bar Wasserstoff 16 h bei 20°C und 600 U/min gerührt. Anschließend wurden weitere 2,5 g (0,22 mmol) 5% Pt/C Katalysator (66% wasserfeucht) zugegeben und unter gleichen Bedingungen weiter hydriert. Danach wurde der Autoklav entspannt, die Reaktionsmischung über eine Nutsche abgesaugt und der Rückstand mit Methanol gewaschen. Das Filtrat wurde unter reduziertem Druck auf 125,7 g eingeengt.

GC/MS (m/z): 219 [M-CH₂O], 204, 190, 160, 146, 132, 120, 105, 91, 82, 79, 77, 69.

¹H-NMR (DMSO-d6, 600 MHz): 2,19-2,28 (1H, m), 2,4 (3H, s), 2,7-2,78 (1H, m), 3,68 (3H, s), 3,9-4,02 (2 H, m), 4,03-4,2 (3H, m), 4,47-4,55 (1H, t), 7,22-7,35 (3H, m), 7,55 (1H, d), 9,6-9,8 (2H, br) ppm.
Reinheit (qNMR): 57%
Ausbeute: 125,7 g der Titelverbindung (73% d. Th.)

### Erfindungsgemäßer Schritt 2

### Beispiel 15

### rac-cis-4-Aminotetrahydrofuran-2-carbonsäuremethylesterhydrochlorid

Zu einer Lösung von 16 g (57,9 mmol) cis-4-(Benzylamino)tetrahydrofuran-2-carbonsäuremethylesterhydrochlorid, 56 g Methanol und 24 g Toluol in einem 300 ml Autoklaven wurden 80 mg (0,025 mmol) 5% Pd/C Katalysator (33% wasserfeucht) gegeben. Der Autoklav wurde dreimal mit 5 bar Argon gespült und dann das Gemisch unter 5 bar Wasserstoff 16 h bei 65°C und 600 U/min gerührt. Der Autoklav wurde auf Raumtemperatur abgekühlt und entspannt. Die Reaktionsmischung wurde über eine Nutsche abgesaugt und das Filtrat unter reduziertem Druck eingeengt und dann das Lösungmittelgemisch auf das Verhältnis Toluol:MeOH=90:10 eingestellt. Die Lösung wurde unter Rühren von 66°C auf Raumtemperatur abgekühlt und die Suspension bei Raumtemperatur über eine Nutsche abgesaugt. Der Rückstand wurde mit je 5 ml Toluol:MeOH=10:1 und Toluol gewaschen und dann unter reduziertem Druck getrocknet.

GC/MS (m/z): 217 [M+TMS], 202, 187, 172, 158, 142, 128, 116, 100, 89, 73, 59, 54.

¹H-NMR (DMSO-d6, 600 MHz): 1,95-2,04 (1H, m), 2,59-2,67 (1H, m), 3,70 (3H, s), 3,75-3,85 (2H, m), 3,9-3,97 (1H, m), 4,45-4,52 (1H, t), 8,2-8,5 (3H, br) ppm.
Reinheit (qNMR): 98%
Ausbeute: 9,86 g der Titelverbindung (92% d. Th.)

### Beispiel 16

### rac-cis-4-Aminotetrahydrofuran-2-carbonsäuremethylesterhydrochlorid

Zu einer Lösung von 125 g (56,9% Reinheit, 249 mmol) cis-4-(2-Methylbenzylamino)tetrahydrofuran-2-carbonsäuremethylesterhydrochlorid und 250 g Methanol in einem 600 ml Autoklaven wurden 2 g (0,94 mmol) 5% Pd/C Katalysator gegeben. Der Autoklav wurde dreimal mit 5 bar Argon gespült und dann das Gemisch unter 5 bar Wasserstoff 16 h bei 65°C und 600 U/min gerührt. Der Autoklav wurde auf Raumtemperatur abgekühlt und entspannt. Die Reaktionsmischung wurde über eine Nutsche abgesaugt, der Rückstand mit Methanol gewaschen und das Filtrat unter reduziertem Druck eingeengt. Der Rückstand wurde mit 22 g Methanol und 182 g Xylol umkristallisiert. Der Kristallbrei wurde über eine Nutsche abgesaugt, mit Xylol gewaschen und unter reduziertem Druck getrocknet.

GC/MS (m/z): 217 [M+TMS], 202, 187, 172, 158, 142, 128, 116, 100, 89, 73, 59, 54.

¹H-NMR (DMSO-d6, 600 MHz): 1,95-2,04 (1H, m), 2,59-2,67 (1H, m), 3,70 (3H, s), 3,75-3,85 (2H, m), 3,9-3,97 (1H, m), 4,45-4,52 (1H, t), 8,2-8,5 (3H, br) ppm.
Reinheit (qNMR): 80%
Ausbeute: 52,4 g der Titelverbindung (93% d. Th.)

## Patentansprüche

1. Verfahren zur Herstellung von Verbindungen der allgemeinen Formel **(I)** oder deren Salze 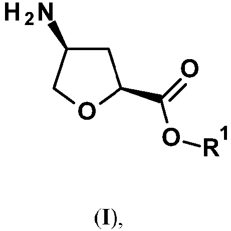 worin
R¹ für (C₁-C₆)Alkyl oder (C₃-C₆)Cycloalkyl steht,
**dadurch gekennzeichnet, dass** in einem ersten Schritt Verbindungen der allgemeinen Formel **(II)** 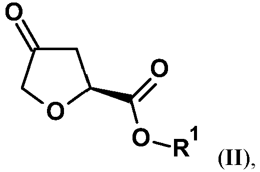 worin
R¹ wie oben definiert ist
mit einem Amin der allgemeinen Formel **(III)** oder deren Salze 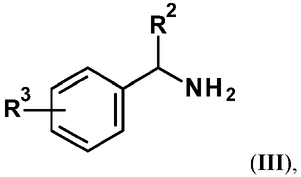 worin
R² für H, (C₁-C₄)Alkyl, unsubstituiertes oder substituiertes Phenyl steht,
R³ für H, Halogen, (C₁-C₄)Alkyl oder (C₁-C₄)Alkoxy steht,
in Gegenwart von H₂ und einem Katalysator in einem Lösungsmittel, ausgewählt aus R¹OH, R'OH/Toluol, 2-PrOH, 2-BuOH oder t-Amylalkohol, wobei R¹ wie oben definiert ist, zu Verbindungen der allgemeinen Formel **(IV)** oder deren Salze reagieren 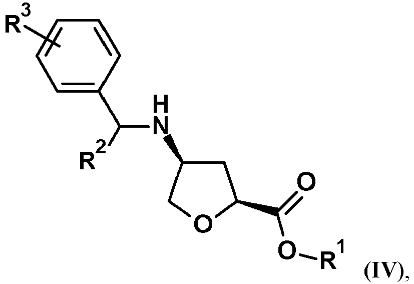
wobei R¹, R² und R³ wie oben definiert sind,
und diese anschließend in Gegenwart von H₂ und einem Katalysator in einem Lösungsmittel, ausgewählt aus R¹OH, R'OH/Toluol, 2-PrOH, 2-BuOH oder t-Amylalkohol, wobei R¹ wie oben definiert ist, zu Verbindungen der allgemeinen Formel **(I)** oder deren Salze umgesetzt werden.

2. Verfahren nach Anspruch 1, **dadurch gekennzeichnet, dass** die Restedefinitionen für die Verbindungen der allgemeinen Formeln **(I), (II), (III)** und **(IV)** oder deren Salze die folgenden sind:
R¹ steht für (C₁-C₆)Alkyl,
R² steht für H, Phenyl, Methyl
R³ steht für H, F in para-Position des Phenylrings, Methyl-O- in para-Position des Phenylrings, Methyl in ortho-Position des Phenylrings.

3. Verfahren nach Anspruch 1, **dadurch gekennzeichnet, dass** die Restedefinitionen für die Verbindungen der allgemeinen Formeln **(I), (II), (III)** und **(IV)** oder deren Salze die folgenden sind:
R¹ steht für CH₃,
R² steht für H,
R³ steht für H oder Methyl in ortho-Position des Phenylrings.

4. Verfahren nach einem der Ansprüche 1 bis 3, **dadurch gekennzeichnet, dass** die Reaktion von Schritt 1 bei 20 bis 40°C durchgeführt wird.

5. Verfahren nach einem der Ansprüche 1 bis 4, **dadurch gekennzeichnet, dass** die Reaktion von Schritt 2 bei 40 bis 80°C durchgeführt wird.

6. Verfahren nach einem der Ansprüche 1 bis 5, **dadurch gekennzeichnet, dass** die Verbindungen der allgemeinen Formeln **(III), (IV)** und **(I)** als Hydrochlorid-Salz vorliegen.

7. Verfahren nach einem der Ansprüche 1 bis 6, **dadurch gekennzeichnet, dass** der Katalysator in Schritt 1 Pt/C ist.

8. Verfahren nach einem der Ansprüche 1 bis 7, **dadurch gekennzeichnet, dass** der Katalysator in Schritt 2 Pd/C ist.

## Claims

1. Process for preparing compounds of the general formula (I) or salts thereof 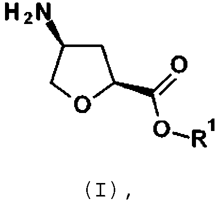 in which
R¹ is (C₁-C₆) alkyl or (C₃-C₆) cycloalkyl,
**characterized in that** in a first step compounds of the general formula (II) 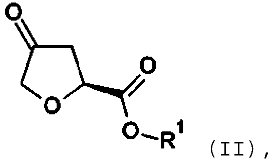
in which
R¹ is as defined above,
react with an amine of the general formula (III) or salts thereof 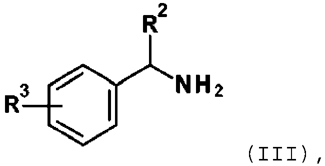
in which
R² is H, (C₁-C₄) alkyl, unsubstituted or substituted phenyl,
R³ is H, halogen, (C₁-C₄) alkyl or (C₁-C₄) alkoxy,
in the presence of H₂ and a catalyst in a solvent, selected from R¹OH, R¹OH/toluene, 2-PrOH, 2-BuOH or *t-*amyl alcohol, where R¹ is as defined above, to form compounds of the general formula (IV) or salts thereof 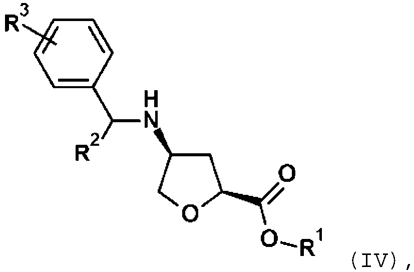
where R¹, R² and R³ are as defined above,
and said compounds are then converted in the presence of H₂ and a catalyst in a solvent, selected from R¹OH, R¹OH/toluene, 2-PrOH, 2-BuOH or t-amyl alcohol, where R¹ is as defined above, into compounds of the general formula (I) or salts thereof.

2. Process according to Claim 1, **characterized in that** the definitions of the radicals for the compounds of the general formulae (I), (II), (III) and (IV) or salts thereof are as follows:
R¹ is (C₁-C₆) alkyl,
R² is H, phenyl, methyl,
R³ is H, F in para position of the phenyl ring, methyl-O- in para position of the phenyl ring, methyl in ortho position of the phenyl ring.

3. Process according to Claim 1, **characterized in that** the definitions of the radicals for the compounds of the general formulae (I), (II), (III) and (IV) or salts thereof are as follows:
R¹ is CH₃,
R² is H,
R³ is H or methyl in ortho position of the phenyl ring.

4. Process according to any of Claims 1 to 3, **characterized in that** the reaction of step 1 is conducted at 20°C to 40°C.

5. Process according to any of Claims 1 to 4, **characterized in that** the reaction of step 2 is conducted at 40°C to 80°C.

6. Process according to any of Claims 1 to 5, **characterized in that** the compounds of the general formulae (III), (IV) and (I) are present as hydrochloride salt.

7. Process according to any of Claims 1 to 6, **characterized in that** the catalyst in step 1 is Pt/C.

8. Process according to any of Claims 1 to 7, **characterized in that** the catalyst in step 2 is Pd/C.

## Revendications

1. Procédé de préparation de composés de formule générale (I) ou leurs sels 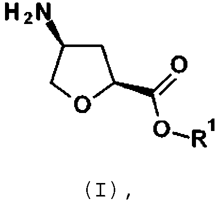 dans laquelle
R¹ représente (C₁-C₆)-alkyle ou (C₃-C₆) -cycloalkyle, **caractérisé en ce que**, dans une première étape, des composés de formule générale (II) 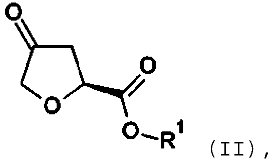 dans laquelle
R¹ est défini comme ci-dessus
réagissent avec une amine de formule générale (III) ou ses sels 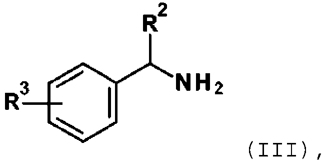
dans laquelle
R² représente H, (C₁-C₄)-alkyle, phényle non substitué ou substitué,
R³ représente H, halogène, (C₁-C₄) -alkyle ou (C₁-C₄) - alcoxy,
en présence de H₂ et d'un catalyseur dans un solvant, choisi parmi R¹OH, R¹OH/toluène, 2-PrOH, 2-BuOH ou alcool t-amylique, R¹ étant défini comme ci-dessus, en composés de formule générale (IV) ou leurs sels 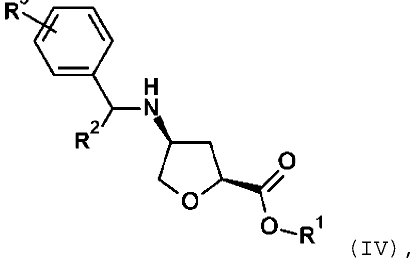
dans laquelle R¹, R² et R³ sont définis comme ci-dessus, et ceux-ci sont ensuite transformés, en présence de H₂ et d'un catalyseur dans un solvant, choisi parmi R¹OH, R¹OH/toluène, 2-PrOH, 2-BuOH ou alcool t-amylique, R¹ étant défini comme ci-dessus, en composés de formule générale (I) ou leurs sels.

2. Procédé selon la revendication 1, **caractérisé en ce que** les définitions des radicaux pour les composés des formules générales (I), (II), (III) et (IV) ou leurs sels sont les suivantes :
R¹ représente (C₁-C₆)-alkyle,
R² représente H, phényle, méthyle
R³ représente H, F en position para du cycle phényle,
méthyl-O- en position para du cycle phényle, méthyle en position ortho du cycle phényle.

3. Procédé selon la revendication 1, **caractérisé en ce que** les définitions des radicaux pour les composés des formules générales (I), (II), (III) et (IV) ou leurs sels sont les suivantes :
R¹ représente CH₃,
R² représente H,
R³ représente H ou méthyle en position ortho du cycle phényle.

4. Procédé selon l'une des revendications 1 à 3, **caractérisé en ce que** la réaction de l'étape 1 est réalisée à 20°C jusqu'à 40°C.

5. Procédé selon l'une des revendications 1 à 4, **caractérisé en ce que** la réaction de l'étape 2 est réalisée à 40°C jusqu'à 80°C.

6. Procédé selon l'une des revendications 1 à 5, **caractérisé en ce que** les composés des formules générales (III), (IV) et (I) se trouvent sous forme de sel de chlorhydrate.

7. Procédé selon l'une des revendications 1 à 6, **caractérisé en ce que** le catalyseur dans l'étape 1 est Pt/Cl.

8. Procédé selon l'une des revendications 1 à 7, **caractérisé en ce que** le catalyseur dans l'étape 2 est Pd/C.
